Europäisches Patentamt

European Patent Office

Office européen des brevets

(19)

(11) Numéro de publication : **0 177 428**
**B1**

(12) **FASCICULE DE BREVET EUROPÉEN**

(45) Date de publication du fascicule du brevet :
17.08.88

(21) Numéro de dépôt : **85401933.8**

(22) Date de dépôt : **03.10.85**

(51) Int. Cl.⁴ : **B 01 J 2/20**, C 07 C 31/26

(54) **Procédé de préparation de mannitol granulaire directement compressible.**

(30) Priorité : **03.10.84 FR 8415212**

(43) Date de publication de la demande :
**09.04.86 Bulletin 86/15**

(45) Mention de la délivrance du brevet :
**17.08.88 Bulletin 88/33**

(84) Etats contractants désignés :
**AT BE CH DE GB IT LI LU NL SE**

(56) Documents cités :
**EP-A- 0 054 868**
**GB-A- 2 054 599**

(73) Titulaire : **Roquette Frères**

**F-62136 Lestrem (FR)**

(72) Inventeur : **Serpelloni, Michel**
**270, Boulevard Voltaire**
**F-62400 Béthune (FR)**
Inventeur : **Lemay, Patrick**
**29, rue du Puits Richebourg**
**F-62136 Lestrem (FR)**

(74) Mandataire : **Koch, Gustave et al**
**Cabinet PLASSERAUD 84, rue d'Amsterdam**
**F-75009 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

L'invention a pour objet un procédé de préparation de mannitol granulaire directement compressible.

Le mannitol, de par ses propriétés, notamment de par sa saveur et son hygroscopicité faible, constitue un excipient pharmaceutique de choix dès lors qu'on le rend directement compressible : or, il ne présente pas naturellement cette propriété notamment lorsqu'il est obtenu par cristallisation dans l'eau.

On trouve déjà, dans le commerce, du mannitol directement compressible sous forme granulaire, avec ou sans liant de granulation.

Lorsqu'il n'y a pas de liant, la compressibilité du produit n'est pas très satisfaisante et la friabilité est en outre trop élevée.

Par ailleurs, la présence d'un liant — qui combat la friabilité et augmente la compressibilité — n'est pas très prisée par l'utilisateur.

Il existait, par conséquent, un besoin de mannitol granulaire directement compressible ne comportant pas de liant et de propriétés de compressibilité et de friabilité améliorées par rapport à celles des produits déjà existants.

C'est ce besoin que la Société Demanderesse s'est efforcé de satisfaire.

Or, elle a eu le mérite de trouver qu'il était possible de préparer un tel mannitol granulaire directement compressible, exempt de liant et de propriétés de friabilité et de compressibilité améliorées, en soumettant du mannitol poudre à un traitement d'extrusion dont les paramètres de température, durée et pression sont choisis de façon telle qu'à la sortie de la zone d'extrusion le mannitol ainsi traité se trouve à l'état partiellement fondu.

En conséquence, le procédé de fabrication du mannitol granulaire directement compressible conforme à l'intervention est caractérisé par le fait qu'une matière première essentiellement constituée de mannitol pourdre est soumise à un traitement d'extrusion à l'intérieur d'une installation comprenant une zone de chauffage à une filière d'extrusion, le débit d'alimentation de l'installation en matière première ainsi que les paramètres du traitement d'extrusion, à savoir la température régnant à l'intérieur de la zone de chauffage, le diamètre de la filière d'extrusion et la vitesse d'entraînement de la matière première à l'intérieur de la zone de chauffage étant sélectionnés de façon telle qu'à la sortie de la filière et avant la sortie du mannitol de cette dernière, ledit mannitol soit partiellement fondu.

Selon un mode de réalisation avantageux du susdit procédé, on traite la matière première à l'intérieur d'une installation d'extrusion du type bi-vis comportant une filière d'extrusion et on sélectionne les paramètres du traitement d'extrusion de façon telle que la matière première se trouve à une température de 160 à 170 °C et, de préférence, de 165 à 168 °C à l'intérieur de la filière et avant la sortie du mannitol de cette dernière.

Dans ces conditions, on estime que la proportion de mannitol fondu est de 30 à 90 %, et plus généralement de 50 à 80 %.

Le temps de passage du mannitol à travers l'installation d'extrusion se situe avantageusement dans une plage de 0,5 à 10 minutes, de préférence de 1 à 4 minutes.

L'invention vise encore d'autres dispositions qui s'utilisent de préférence en même temps et dont il sera plus explicitement question ci-après et elle pourra, de toute façon, être bien comprise à l'aide du complément de description qui suit ainsi que du dessin annexé et de l'exemple, lesdits complément de description et exemple étant relatifs à des modes de réalisation avantageux.

La figure 1 du dessin représente, en coupe schématique, une installation d'extrusion du type de celles pouvant être utilisées dans le cadre du procédé selon l'invention.

La figure 2 est une coupe diamétrale cotée d'un comprimé préparé à partir de mannitol compressible, ce comprimé servant à effectuer certaines mesures caractérisant la compressibilité du mannitol obtenu conformément à l'invention ainsi que celle des mannitols compressibles de l'art antérieur.

Se proposant, par conséquent, de fabriquer, conformément à l'invention, du mannitol granulaire directement compressible sans intervention d'un liant et présentant une friabilité abaissée par rapport à celle du mannitol directement compressible de l'art antérieur, on s'y prend comme suit ou de façon équivalente.

La matière première qui est soumise au traitement d'extrusion est constituée de mannitol poudre essentiellement sous forme de mannitol cristallisé.

Dans la pratique, on utilise des cristaux natifs de mannitol, c'est-à-dire des cristaux obtenus de façon conventionnelle par cristallisation dans l'eau ; dans la pratique également, on ajoute à ces cristaux natifs, par recyclage, la partie du mannitol, obtenue à l'issue du procédé, qui n'est pas utilisable commercialement, par exemple en raison d'une granulométrie trop faible.

Les cristaux de mannitol sont introduits dans une installation d'extrusion sous forme de poudre, éventuellement en présence d'une faible quantité d'eau inférieure à 4 % et, de préférence, inférieure à 2 %.

La température de ces cristaux est généralement comprise, à l'entrée de l'installation d'extrusion, entre 5 et 80 °C sans que cela constitue un facteur limitatif de l'invention.

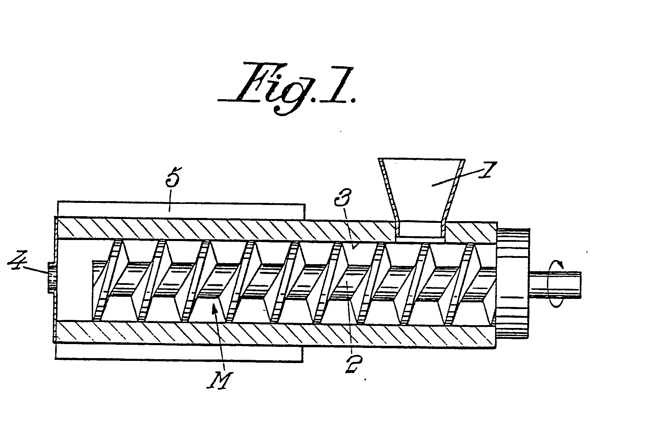

L'installation d'extrusion est constituée avantageusement par une installation, ou extrudeuse, du type bi-vis comprenant, comme montré à la figure 1 :
— un système d'alimentation, notamment une trémie doseuse et mélangeuse 1,
— une zone de malaxage M comprenant un système à deux vis sans fin 2 disposées à l'intérieur d'un carter 3, notamment en acier nitruré, et entraînées en rotation par un mécanisme non montré.
— une sortie comprenant une ou plusieurs filières 4 de différentes formes.
— des moyens de chauffage 5 permettant de contrôler la température de la zone de malaxage, ces moyens de chauffage 5 étant constitués par exemple par des résistances électriques, par un système de chauffage par induction ou à la vapeur et par des moyens de refroidissement non montrés disposés à l'extérieur du carter ou à l'intérieur et se présentant par exemple sous la forme de serpentins logés dans le carter, d'un circuit de fluide de refroidissement logé à l'intérieur de la vis, et autres.

La matière première entrant par l'organe d'alimentation dans la zone de malaxage est soumise, grâce à la compression réalisée dans les spires de la vis, à un cisaillement et à un frottement mécanique intenses simultanément à l'échauffement induit par l'élément chauffant.

L'extrusion constitue, par conséquent, un traitement thermo-mécanique.

Pour fixer les idées, on signale qu'on a obtenu de bons résultats avec une extrudeuse du type bi-vis mise sur la marche sous la dénomination « BC 82 » par la Société CREUSOT-LOIRE. Les deux vis se copénètrent et tournent dans le même sens. La zone de malaxage est chauffée par induction et la température peut donc y être facilement régulée.

L'avantage essentiel de ce mode de chauffage est sa souplesse d'utilisation et son contrôle aisé au moyen d'une boucle de régulation simple (thermocouple/dispositif de commande de l'alimentation électrique des moyens de chauffage par induction). Il se pourrait que l'existence d'un champ électromagnétique intense exerce une influence sur les propriétés du produit.

Dans le cas de l'installation utilisée dans le cadre de l'exemple décrit plus loin, la filière utilisée était en forme cylindrique et avait un diamètre de 3 mm.

La température de la zone de chauffage est obtenue en imposant au système de chauffage une valeur prédéterminée. Dans le cas de l'installation d'extrusion dont il vient d'être question, cette valeur est comprise entre 230 et 300 °C de préférence entre 260 à 300 °C et, plus préférentiellement encore, voisine de 280 °C.

Les caractéristiques mécaniques des vis et leur vitesse de rotation sont choisies de façon telle que la durée du séjour de la matière première à l'intérieur de la zone de chauffage soit voisine de 2 minutes.

Grâce au choix de l'ensemble de ces paramètres, la température de la matière première ayant subi le traitement, est de 165 à 168 °C à l'intérieur de la filière et avant sa sortie de cette dernière.

Le mannitol obtenu à la sortie de l'installation d'extrusion est successivement soumis :
— à un refroidissement,
— à un broyage,
— à un tamisage et
— à un recyclage des fines (particules de taille trop faible pour être retenues par le plus petit tamis de l'installation) au niveau de l'alimentation de l'extrudeuse.

Avant de tester les performances en compression du mannitol ainsi obtenu, on lui fait comprendre un agent lubrifiant, en l'occurrence par exemple du stéarate de magnésium.

On donne ci-après un exemple illustrant le procédé.

Exemple

Du mannitol cristallisé d'une pureté chimique supérieure à 98 % et une teneur en eau de 0,07 %, est utilisé pour alimenter une extrudeuse «BC 82» de la société CREUSOT-LOIRE du type décrit ci-dessus.

La vitesse des vis est réglée de manière à ce que le débit de l'installation soit de 250 kg/heure et que le temps de contact soit de 2 minutes.

La température de consigne du système de chauffage est programmée à 270 °C, ce qui permet d'obtenir à la sortie de l'extrudeuse du mannitol à une température de 166 °C.

Ce mannitol se présente sous la forme de bâtonnets qui sont broyés à l'aide d'une broyeuse du type à marteaux.

Par tamisage, on sépare les fines qui sont recyclées au niveau de l'alimentation de l'extrudeuse.

Pour montrer l'avantage qu'il y a à utiliser, dans la fabrication de mannitol directement compressible, le procédé conforme à l'invention, on a procédé aux mesures et essais comparatifs qui vont être décrits.

Ces essais portent :
— sur le mannitol granulaire directement compressible selon l'exemple et, à titre de comparaison,
— sur un premier mannitol granulaire directement compressible du commerce sans liant, ainsi que
— sur un deuxième mannitol granulaire directement compressible comportant 1,5 % en poids d'un liant constitué de gélatine.

On détermine tout d'abord la répartition granulométrique et la granulométrie moyenne (correspondant à 50 % de la distribution en masse) des différentes poudres de mannitol soumises aux essais comparatifs et notamment au test de compression.

On mesure également l'indice d'écoulement de ces poudres en ayant recours à la méthode de CARR telle que décrite par CARR R.L. dans Chem. Eng. 72, N° 1, 163, 168, (1965) et Chem. Eng. 72, N° 2, 69-

3

# 0 177 428

73 (1965) ; on utilise, pour ce faire, un appareil connu sous la marque «HOSOKAWA POWDER TESTER» et fabriqué par MICROMERITICS, Osaka (Japon).

On détermine également la friabilité de ces poudres. Cette propriété est caractérisée comme étant représentée par le pourcentage de particules n'ayant pas résisté à un concassage dans un appareil appelé friabilimètre. En l'occurrence, on a utilisé celui de marque «ARWEKA TA». Cet appareil contient 5 billes d'acier identiques de 1,7 cm de diamètre et de 18,87 g chacune.

On y introduit 15 g d'une fraction granulométrique de 400 à 500 microns de la poudre testée et on met l'appareil en rotation de 25 tours/minute pendant 15 minutes.

On détermine par pesée, à la fin du concassage, la proportion, exprimée en %, que représente le résidu retenu par un tamis de largeur de maille de 351 microns ; la valeur de la friabilité correspond au complément à 100 de cette dernière valeur. Plus le chiffre ainsi obtenu est grand, plus la friabilité est grande ; il est rappelé que l'on recherche les poudres de faible friabilité.

Enfin, on détermine la compressibilité de ces différentes poudres ; cette compressibilité est traduite en termes de «résistance à la rupture», exprimée en newton et mesurée sur des comprimés préparés à l'aide des diverses poudres de mannitol.

Les comprimés sont fabriqués sous une pression de 196,133 MPa dans une presse rotative à haut rendement du type P 1000 fabriquée par la Société WILHELM FETTE GmbH (2503 Schwarzen-beck — R.F.A.) et équipée des jauges de mesure des forces de compression et d'éjection.

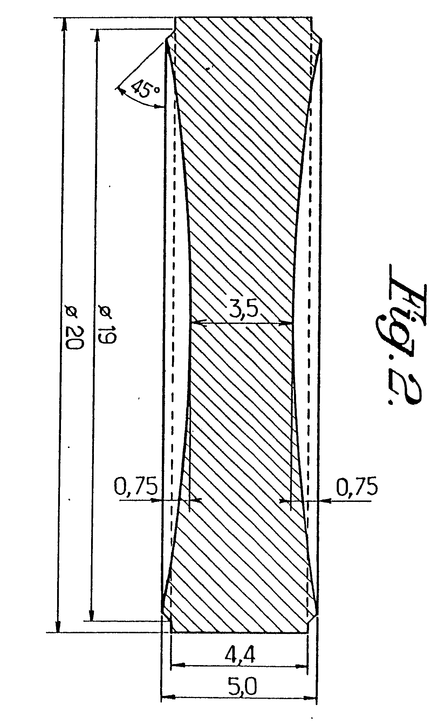

Ces comprimés sont ronds, biconcaves et se présentent sous la forme et avec les dimensions résultant de la figure 2.

La compressibilité est exprimée, comme indiqué plus haut, par la valeur en newton de la résistance à la rupture, mesurée sur les comprimés à l'aide d'un duromètre ERWEKA TB 24, équipé d'un chevalet de support de tablettes ayant une distance entre appuis de 10,55 mm (cet appareil est construit par ERWEKA APPARATEBAU GmbH — 6056 Heusenstamm — R.F.A.).

Les résultats de ces mesures ainsi que la répartition granulométrique, la granulométrie moyenne, la teneur en lubrifiant constitué par du stéarate de magnésium, les indices d'écoulement et la friabilité des diverses poudres sont réunis dans le tableau ci-après.

(Voir Tableau page 5)

4

Tableau

| | Mannitol selon l'invention (exemple) | Mannitol directement compressible du commerce sans liant | Mannitol directement compressible avec liant (1,5% de gélatine) |
|---|---|---|---|
| Répartition granulo-  ) > 1000 µm<br>métrique en %        ) >  500 µm<br>cumulé en masse       ) >  250 µm | 3<br>77<br>99 | 8,1<br>95<br>99 | 3<br>77<br>99 |
| Granulométrie moyenne (en µm) | 620 | 860 | 650 |
| Teneur en % de stéarate de magnésium | 0,5 | 0,8 | 1 |
| Résistance à la rupture (en newton) | 114,7 | 59,8 | 130,4 |
| Friabilité (en %) | 75 | 81 | 70 |
| Ecoulement (indice de Carr) | 85 | 82 | 85 |

0 177 428

**0 177 428**

Il résulte de ce tableau que le mannitol suivant l'invention présente une friabilité intermédiaire entre celle du mannitol commercial (sans liant) et celle du manitol (avec liant) et qu'il présente une compressibilité très proche de celle du mannitol avec liant et très supérieure à celle du mannitol commercial sans liant.

Il présente en outre un indice d'écoulement libre suffisamment élevé — plus précisément égal à celui du mannitol comportant un liant — pour être exploité commercialement comme un excipient de compression directe.

Enfin, toutes ces valeurs sont atteintes pour une granulométrie moyenne plus basse que celle du mannitol connu sans liant et même plus faible que celle du mannitol avec liant, ce qui est précieux pour la fabrication de comprimés pharmaceutiques faiblement dosés en principes actifs.

Le mannitol obtenu par mise en oeuvre du procédé selon l'invention sera donc avantageusement utilisé en remplacement des mannitols directement compressibles actuellement sur le marché qui lorsqu'ils ne comportent pas de liant, présentent une trop faible compressibilité et une trop grande friabilité.

Comme il va de soi et comme il résulte d'ailleurs déjà de ce qui précède, l'invention ne se limite nullement à ceux de ses modes d'application et de réalisation qui ont été plus spécialement envisagés ; elle en embrasse, au contraire, toutes les variantes.

## Revendications

1. Procédé de préparation de mannitol granulaire directement compressible, caractérisé par le fait qu'une matière première essentiellement constituée de mannitol poudre est soumise à un traitement d'extrusion à l'intérieur d'une installation comprenant une zone de chauffage et une filière d'extrusion, le débit d'alimentation de l'installation en matière première ainsi que le paramètres du traitement d'extrusion, à savoir la température régnant à l'intérieur de la zone de chauffage, le diamètre de la filière d'extrusion et la vitesse d'entraînement de la matière première à l'intérieur de la zone de chauffage étant sélectionnés de façon telle qu'à la sortie de la filière et avant la sortie du mannitol de cette dernière, ledit mannitol soit partiellement fondu.

2. Procédé selon la revendication 1, caractérisé par le fait que l'on traite la matière première à l'intérieur d'une installation d'extrusion, de préférence du type bi-vis, comportant une filière d'extrusion et on sélectionne les paramètres du traitement d'extrusion de façon telle que le mannitol se trouve à une température de 160 à 170 °C, de préférence de 165 à 168 °C, à l'intérieur de la filière et avant sa sortie de celle-ci.

3. Procédé selon l'une des revendications 1 à 2, caractérisé par le fait qu'à la sortie de la filière, la proportion de mannitol fondu est de 30 à 90 % et, plus généralement, de 50 à 80 %.

4. Procédé selon l'une des revendications 1 à 3, caractérisé par le fait que la température de la zone de chauffage est obtenue en imposant au système de chauffage comporté par l'installation d'extrusion une température comprise entre 230 et 300 °C, de préférence entre 260 et 300 °C.

## Claims

1. Method of preparing directly compressible granular mannitol, characterized by the fact that a raw material essentially constituted of powder mannitol is subjected to an extrusion treatment inside an installation comprising a heating zone and an extrusion die, the supply flow rate of the installation with raw material as well as the parameters of the extrusion treatment, namely the temperature existing inside the heating zone, the diameter of the extrusion die and the driving speed of the raw material inside the heating zone being selected so that at the exit of the die and before the exit of the mannitol from the latter, said mannitol is partly fused.

2. Method according to Claim 1, characterized by the fact that the raw material is treated inside an extrusion installation, preferably of the dual-screw type, comprising an extrusion die, the parameters of the extrusion treatment being selected so that the mannitol is at a temperature of 160 to 170 °C, preferably from 165 to 168 °C inside the die and before its exit from the latter.

3. Method according to one of Claims 1 and 2, characterized by the fact that at the exit of the die, the proportion of fused mannitol is from 30 to 90 %, more generally, from 50 to 80 %.

4. Method according to one of Claims 1 to 3, characterized by the fact that the temperature of the heating zone is obtained by imposing on the heating system included by the extrusion installation a temperature comprised between 230 and 300 °C, preferably between 260 and 300 °C.

## Patentansprüche

1. Verfahren zur Herstellung von direkt komprimierbarem, körnigem Mannit, dadurch gekennzeichnet, daß ein im wesentlichen aus pulverförmigem Mannit bestehendes Ausgangsmaterial einer Srang-

6

preßbehandlung im Inneren einer Vorrichtung unterworfen wird, welche eine Heizzone und eine Extrusionsdüse umfaßt, wobei die in die Vorrichtung eingeführte Menge an Ausgangsmaterial sowie die Parameter der Strangpreßbehandlung, nämlich die im Inneren der Heizzone herrschende Temperatur, der Durchmesser der Extrusionsdüse und die Geschwindigkeit, mit der das Ausgangsmaterial im Inneren der Heizzone geführt wird, derart ausgewählt werden, daß der genannte Mannit am Ausgang der Extrusionsdüse, und noch bevor er aus dieser letzeren ausgetreten ist, teilweise geschmolzen ist.

2. Verfahren nach Anspruch 1, dadurch gekennzeichnet, daß man das Ausgangsmaterial im Inneren einer eine Extrsiondüse aufweisenden Strangpreßvorrichtung, vorzugsweise einer solchen vom Doppelschneckentypus, behandelt, und daß man die Extrusionsparameter derart auswählt, daß die Temperatur des Mannits im Inneren der Extrusionsdüse und vor seinem Austritt aus derselben 160 bis 170 °C, vorzugsweise 165 bis 168 °C, beträgt.

3. Verfahren nach Anspruch 1 oder 2, dadurch gekennzeichnet, daß der Anteil des geschmolzenen Mannits am Ausgang der Extrusionsdüse 30 bis 90 %, insbesondere 50 bis 80 %, beträgt.

4. Verfahren nach einen der Ansprüche 1 bis 3, dadurch gekennzeichnet, daß die Temperatur der Heizzone dadurch erhalten wird, daß in dem in der Strangpreßvorrichtung enthaltenen Heizsystem eine Temperatur aufrecht erhalten wird, welche zwischen 230 und 300 °C, vorzugsweise zwischen 260 und 300 °C, liegt.

# Fig.1.

# Fig.2.